# EUROPEAN PATENT APPLICATION

(11) **EP 1 210 875 A1**
(43) Date of publication of application: **05.06.2002**
(21) Application number: 00126632.9
(22) Date of filing: 04.12.2000
(51) Int. Cl.: A01K 67/033, C12N 5/10, C12N 15/86

(54) **Modulating gene expression in insects by using double-stranded RNA (dsRNA)**

(71) Applicant: Aventis CropScience GmbH, 65929 Frankfurt am Main (DE)
(72) Inventor: Gunkel, Nikolas, Dr., 69121 Heidelberg (DE)

(57) **Abstract**

The invention relates to the identification of target molecules arthropods for insecticides or acaricides. Target genes of interest encoding such target molecules are especially genes that are active/inactive or show at least different expression profiles especially according to specific developmental stages/life cycles. The identification of these new insecticidal target sites is practised by using double-stranded RNA (dsRNA) molecules for targeting specific genes in transgenic flies.

SEQUENCE LISTING

<110> Aventis CropScience GmbH

<120> Identification of insecticidal target molecules by
      using double-stranded RNA (dsRNA)

<130> AGR 2000/M 237

<140> -
<141> 2000-12-04

<160> 7

<170> PatentIn Ver. 2.1

<210> 1
<211> 71
<212> DNA
<213> Artificial Sequence

<220>
<223> Description of Artificial Sequence:-

<400> 1
tatctcgagc gaaattaata cgactcacta tagggagacc acaacggttt ccctctagtt 60
tattgctagc t                                                      71

<210> 2
<211> 77
<212> DNA
<213> Artificial Sequence

<220>
<223> Description of Artificial Sequence:-

<400> 2
tcgaagctag caataaacta gagggaaacc gttgtggtct ccctatagtg agtcgtatta 60
atttcgctcg agatatg                                                77

<210> 3
<211> 68
<212> DNA
<213> Artificial Sequence

<220>
<223> Description of Artificial Sequence:-

<400> 3
aacaggcctt taatacgact cactataggg agaccacaac ggtttccctc tactttattg 60
cggccgca 68

<210> 4
<211> 72
<212> DNA
<213> Artificial Sequence

<220>
<223> Description of Artificial Sequence:-

<400> 4
gatctgcggc cgcaataaag tagagggaaa ccgttgtggt ctccctatag tgagtcgtat 60
taaaggcctg tt 72

<210> 5
<211> 2467
<212> DNA
<213> Artificial Sequence

<220>
<223> Description of Artificial Sequence:-

<400> 5
agattgtact gagagtgcac catatctcga gcgaaattaa tacgactcac tatagggaga 60
ccacaacggt ttccctctag tttattgcta gcttcgagca gctgaagctt gcatgcctgc 120
aggtcgactc tagaggatcc ccgggtaccg agctcgaatt catcgatgat atcagatctg 180
cggccgcaat aaagtagagg gaaaccgttg tggtctccct atagtgagtc gtattaaagg 240
cctgttaacc tgcattaatg aatcggccaa cgcgcgggga gaggcggttt gcgtattggg 300
cgctcttccg cttcctcgct cactgactcg ctgcgctcgg tcgttcggct gcggcgagcg 360
gtatcagctc actcaaaggc ggtaatacgg ttatccacag aatcagggga taacgcagga 420
aagaacatgt gagcaaaagg ccagcaaaag gccaggaacc gtaaaaaggc cgcgttgctg 480
gcgtttttcc ataggctccg cccccctgac gagcatcaca aaaatcgacg ctcaagtcag 540

aggtggcgaa acccgacagg actataaaga taccaggcgt ttccccctgg aagctccctc 600
gtgcgctctc ctgttccgac cctgccgctt accggatacc tgtccgcctt tctcccttcg 660
ggaagcgtgg cgctttctca atgctcacgc tgtaggtatc tcagttcggt gtaggtcgtt 720
cgctccaagc tgggctgtgt gcacgaaccc cccgttcagc ccgaccgctg cgccttatcc 780
ggtaactatc gtcttgagtc caacccggta agacacgact tatcgccact ggcagcagcc 840
actggtaaca ggattagcag agcgaggtat gtaggcggtg ctacagagtt cttgaagtgg 900
tggcctaact acggctacac tagaaggaca gtatttggta tctgcgctct gctgaagcca 960
gttaccttcg gaaaaagagt tggtagctct tgatccggca aacaaaccac cgctggtagc 1020
ggtggttttt ttgtttgcaa gcagcagatt acgcgcagaa aaaaaggatc tcaagaagat 1080
cctttgatct tttctacggg gtctgacgct cagtggaacg aaaactcacg ttaagggatt 1140
ttggtcatga gattatcaaa aaggatcttc acctagatcc ttttaaatta aaaatgaagt 1200
tttaaatcaa tctaaagtat atatgagtaa acttggtctg acagttacca atgcttaatc 1260
agtgaggcac ctatctcagc gatctgtcta tttcgttcat ccatagttgc ctgactcccc 1320
gtcgtgtaga taactacgat acgggagggc ttaccatctg gccccagtgc tgcaatgata 1380
ccgcgagacc cacgctcacc ggctccagat ttatcagcaa taaaccagcc agccggaagg 1440
gccgagcgca gaagtggtcc tgcaacttta tccgcctcca tccagtctat taattgttgc 1500
cgggaagcta gagtaagtag ttcgccagtt aatagtttgc gcaacgttgt tgccattgct 1560
acaggcatcg tggtgtcacg ctcgtcgttt ggtatggctt cattcagctc cggttcccaa 1620
cgatcaaggc gagttacatg atcccccatg ttgtgcaaaa aagcggttag ctccttcggt 1680
cctccgatcg ttgtcagaag taagttggcc gcagtgttat cactcatggt tatggcagca 1740
ctgcataatt ctcttactgt catgccatcc gtaagatgct tttctgtgac tggtgagtac 1800
tcaaccaagt cattctgaga atagtgtatg cggcgaccga gttgctcttg cccggcgtca 1860
atacgggata ataccgcgcc acatagcaga actttaaaag tgctcatcat tggaaaacgt 1920
tcttcggggc gaaaactctc aaggatctta ccgctgttga gatccagttc gatgtaaccc 1980
actcgtgcac ccaactgatc ttcagcatct tttactttca ccagcgtttc tgggtgagca 2040
aaaacaggaa ggcaaaatgc cgcaaaaaag ggaataaggg cgacacggaa atgttgaata 2100
ctcatactct tcctttttca atattattga agcatttatc agggttattg tctcatgagc 2160
ggatacatat ttgaatgtat ttagaaaaat aaacaaatag gggttccgcg cacatttccc 2220
cgaaaagtgc cacctgacgt ctaagaaacc attattatca tgacattaac ctataaaaat 2280
aggcgtatca cgaggccctt tcgtctcgcg cgtttcggtg atgacggtga aaacctctga 2340
cacatgcagc tcccggagac ggtcacagct tgtctgtaag cggatgccgg gagcagacaa 2400
gcccgtcagg gcgcgtcagc gggtgttggc gggtgtcggg gctggcttaa ctatgcggca 2460
tcagagc 2467

<210> 6
<211> 8486
<212> DNA
<213> Artificial Sequence

<220>
<223> Description of Artificial Sequence:-

<400> 6
ttcaatgata tccagtgcag taaaaaataa aaaaaaaata tgttttttta aatctacatt 60
ctccaaaaaa gggttttatt aacttacata catactagaa ttaattcctc gagcgaaatt 120
aatacgactc actataggga gaccacaacg gtttccctct agtttattgc tagcttcgag 180
cagctgaagc ttgcatgcct gcaggtcgac tctagaggat ccccgggtac cgagctcgaa 240
ttcatcgatg atatcagatc tgcggccgca ataaagtaga gggaaaccgt tgtggtctcc 300
ctatagtgag tcgtattaaa ggcctaattc cagctgagcg ccggtcgcta ccattaccag 360
ttggtctggt gtcggggatc cgtcgactaa ggccaaagag tctaattttt gttcatcaat 420
gggttataac atatgggtta tattataagt ttgttttaag tttttgagac tgataagaat 480
gtttcgatcg aatattccat agaacaacaa tagtattacc taattaccaa gtcttaattt 540
agcaaaaatg ttattgctta tagaaaaaat aaattattta tttgaaattt aaagtcaact 600
tgtcatttaa tgtcttgtag acttttgaaa gtcttacgat acattagtat ctatatacat 660
ggttcattct acattctata ttagtgatga tttctttagc tagtaataca ttttaattat 720
attcggcttt gatgattttc tgattttttc cgaacggatt ttcgtagacc ctttcgatct 780
cataatggct cattttattg cgatggacgg tcaggagagc tcgaattaac ggggatccgt 840
cgacctgcag cccaagcttt gcgtactcgc aaattattaa aaataaaact ttaaaaataa 900
tttcgtctaa ttaatattat gagttaattc aaaccccacg gacatgctaa gggttaatca 960
acaatcatat cgctgtctca ctcagactca atacgacact cagaatacta ttcctttcac 1020
tcgcacttat tgcaagcata cgttaagtgg atgtctcttg ccgacgggac caccttatgt 1080
tatttcatca tggtctggcc attctcatcg tgagcttccg ggtgctcgca tatctggctc 1140
taagacttcg ggcccgacgc aaggagtagc cgacatatat ccgaaataac tgcttgtttt 1200
tttttttacc attattacca tcgtgtttac tgtttattgc cccctcaaaa agctaatgta 1260
attatatttg tgccaataaa aacaagatat gacctataga atacaagtat ttccccttcg 1320
aacatcccca caagtagact ttggatttgt cttctaacca aaagacttac acacctgcat 1380
accttacatc aaaaactcgt ttatcgctac ataaaacacc gggatatatt ttttatatac 1440
atacttttca aatcgcgcgc cctcttcata attcacctcc accacaccac gtttcgtagt 1500
tgctctttcg ctgtctccca cccgctctcc gcaacacatt caccttttgt tcgacgacct 1560
tggagcgact gtcgttagtt ccgcgcgatt cggtgcggta tttcacaccg catatggtgc 1620
actctcagta caatctgctc tgatgccgca tagttaagcc agccccgaca cccgccaaca 1680
cccgctgacg cgccctgacg ggcttgtctg ctcccggcat ccgcttacag acaagctgtg 1740
accgtctccg ggagctgcat gtgtcagagg ttttcaccgt catcaccgaa acgcgcgaga 1800
cgaaagggcc tcgtgatacg cctattttta taggttaatg tcatgataat aatggtttct 1860
tagacgtcag gtggcacttt tcggggaaat gtgcgcggaa cccctatttg tttatttttc 1920
taaatacatt caaatatgta tccgctcatg agacaataac cctgataaat gcttcaataa 1980

tattgaaaaa ggaagagtat gagtattcaa catttccgtg tcgcccttat tccctttttt 2040
gcggcatttt gccttcctgt ttttgctcac ccagaaacgc tggtgaaagt aaaagatgct 2100
gaagatcagt tgggtgcacg agtgggttac atcgaactgg atctcaacag cggtaagatc 2160
cttgagagtt ttcgccccga agaacgtttt ccaatgatga gcacttttaa agttctgcta 2220
tgtggcgcgg tattatcccg tattgacgcc gggcaagagc aactcggtcg ccgcatacac 2280
tattctcaga atgacttggt tgagtactca ccagtcacag aaaagcatct tacggatggc 2340
atgacagtaa gagaattatg cagtgctgcc ataaccatga gtgataacac tgcggccaac 2400
ttacttctga caacgatcgg aggaccgaag gagctaaccg cttttttgca caacatgggg 2460
gatcatgtaa ctcgccttga tcgttgggaa ccggagctga atgaagccat accaaacgac 2520
gagcgtgaca ccacgatgcc tgtagcaatg gcaacaacgt tgcgcaaact attaactggc 2580
gaactactta ctctagcttc ccggcaacaa ttaatagact ggatggaggc ggataaagtt 2640
gcaggaccac ttctgcgctc ggcccttccg gctggctggt ttattgctga taaatctgga 2700
gccggtgagc gtgggtctcg cggtatcatt gcagcactgg ggccagatgg taagccctcc 2760
cgtatcgtag ttatctacac gacggggagt caggcaacta tggatgaacg aaatagacag 2820
atcgctgaga taggtgcctc actgattaag cattggtaac tgtcagacca agtttactca 2880
tatatacttt agattgattt aaaacttcat ttttaattta aaaggatcta ggtgaagatc 2940
ctttttgata atctcatgac caaaatccct taacgtgagt tttcgttcca ctgagcgtca 3000
gaccccgtag aaaagatcaa aggatcttct tgagatcctt tttttctgcg cgtaatctgc 3060
tgcttgcaaa caaaaaaacc accgctacca gcggtggttt gtttgccgga tcaagagcta 3120
ccaactcttt ttccgaaggt aactggcttc agcagagcgc agataccaaa tactgtcctt 3180
ctagtgtagc cgtagttagg ccaccacttc aagaactctg tagcaccgcc tacatacctc 3240
gctctgctaa tcctgttacc agtggctgct gccagtggcg ataagtcgtg tcttaccggg 3300
ttggactcaa gacgatagtt accggataag gcgcagcggt cgggctgaac ggggggttcg 3360
tgcacacagc ccagcttgga gcgaacgacc tacaccgaac tgagatacct acagcgtgag 3420
cattgagaaa gcgccacgct tcccgaaggg agaaaggcgg acaggtatcc ggtaagcggc 3480
agggtcggaa caggagagcg cacgagggag cttccagggg gaaacgcctg gtatctttat 3540
agtcctgtcg ggtttcgcca cctctgactt gagcgtcgat ttttgtgatg ctcgtcaggg 3600
gggcggagcc tatggaaaaa cgccttcttc ttgaactcgg gctcggtgcc agtatacctc 3660
aaatggttgt cgtacctctc atggttccgt tacgccaacg agggtctgct gattaaccaa 3720
tgggcggacg tggagccggg cgaaattagc tgcacatcgt cgaacaccac gtgccccagt 3780
tcgggcaagg tcatcctgga gacgcttaac ttctccgccg ccgatctgcc gctggactac 3840
gtgggtctgg cccatgatga aataacataa ggtggtcccg tcgatagccg aagcttaccg 3900
aagtatacac ttaaattcag tgcacgtttg cttgttgaga ggaaaggttg tgtgcggacg 3960
aatttttttt tgaaaacatt aacccttacg tggaataaaa aaaaatgaaa tattgcaaat 4020
tttgctgcaa agctgtgact ggagtaaaat taattcacgt gccgaagtgt gctattaaga 4080
gaaaattgtg ggagcagagc cttgggtgca gccttggtga aaactcccaa atttgtgata 4140
cccactttaa agattcgcag tggaaggctg cacctgcaaa aggtcagaca tttaaaagga 4200
ggcgactcaa cgcagatgcc gtacctagta aagtgataga gcctgaacca gaaaagataa 4260
aagaaggcta taccagtggg agtacacaaa cagagtaagt ttgaatagta aaaaaaatca 4320
tttatgtaaa caataacgtg actgtgcgtt aggtcctgtt cattgtttaa tgaaaataag 4380
agcttgaggg aaaaaattcg tactttggag tacgaaatgc gtcgtttaga gcagcagccg 4440
aattaattct agttccagtg aaatccaagc attttctaaa ttaaatgtat tcttattatt 4500

atagttgtta tttttgatat atataaacaa cactattatg cccaccattt ttttgagatg 4560
catctacaca aggaacaaac actggatgtc actttcagtt caaattgtaa cgctaatcac 4620
tccgaacagg tcacaaaaaa ttaccttaaa aagtcataat attaaattag aataaatata 4680
gctgtgaggg aaatatatac aaatatattg gagcaaataa attgtacata caaatattta 4740
ttactaattt ctattgagac gaaatgaacc actcggaacc atttgagcga accgaatcgc 4800
gcggaactaa cgacagtcgc tccaaggtcg tcgaacaaaa ggtgaatgtg ttgcggagag 4860
cgggtgggag acagcgaaag agcaactacg aaacgtggtg tggtggaggt gaattatgaa 4920
gagggcgcgc gatttgaaaa gtatgtatat aaaaaatata tcccggtgtt ttatgtagcg 4980
ataaacgagt ttttgatgta aggtatgcag gtgtgtaagt cttttggtta gaagacaaat 5040
ccaaagtcta cttgtgggga tgttcgaagg ggaaatactt gtattctata ggtcatatct 5100
tgtttttatt ggcacaaata taattacatt agctttttga gggggcaata aacagtaaac 5160
acgatggtaa taatggtaaa aaaaaaaaca agcagttatt tcggatatat gtcggctact 5220
ccttgcgtcg ggcccgaagt cttagagcca gatatgcgag cacccggaag ctcacgatga 5280
gaatggccag acccacgtag tccagcggca gatcggcggc ggagaagtta agcgtctcca 5340
ggatgacctt gcccgaactg gggcacgtgg tgttcgacga tgtgcagcta atttcgcccg 5400
gctccacgtc cgcccattgg ttaatcagca gaccctcgtt ggcgtaacgg aaccatgaga 5460
ggtacgacaa ccatttgagg tatactggca ccgagcccga gttcaagaag aagccgccaa 5520
agagcaggaa tggtatgata accggcggac ccacagacag cgccatcgag gtcgaggagc 5580
tggcgcagga tattagatat ccgaaggacg ttgacacatt ggccaccaga gtgaccagcg 5640
ccaggcagtt gaagaagtgc agcactccgg cccgcagtcc gatcatcgga taggcaatcg 5700
ccgtgaagac cagtggcact gtgagaaaaa gcggtaattc ggcaatcgtt ttgcccagaa 5760
agtatgtgtc acagcgataa agtcgacttc gggcctccct cataaaaact ggcagctctg 5820
aggtgaacac ctaaatcgaa tcgattcatt agaaagttag taaattatta atatgcaaat 5880
gtattctaaa caagacttac atttatcgtg gcaaagacgt tttgaaaggt catgttggtc 5940
aggaagagga agatggctcc gttgatattc atcacgccca cttgcgtgag ttgttggccc 6000
aaaaagatga ggccaatcaa gatggcaacc atctgcaaat taaaatgtta ctcgcatctc 6060
attaatattc atatcttcaa catgttcgcg agttaaatga aatttattta ttttctgcaa 6120
aactataaac tatacatctc attgaaaaaa actaagaagg gtgtggaatc aggcaattct 6180
aactaaaatc tagcgaattt gtttccaaga attgtaagcg ttatatcatt tgtttccact 6240
ggaaccactc accgttgtct gaataagtcg cacttttacg aggagtggtt ccttgagcac 6300
cgacagccag gatcgccaca ggaccgcccg gaactgcatg aaccaggtgg ccttgtaggt 6360
gtacccattc tccggctgct ccagtggctt ctccaaattt ttggtggcca acaactgctc 6420
catatcccgg gctactttgc taatagcaaa attgtcgcca tatcttggcg atccgatcac 6480
gggactcgat ctcccgtccg ggcacaacgg ccaacacctg tacgtaaaag tccgccggat 6540
tgtagttggt aggacactgg gcacccacgc tggataggag ttgagatgta atgtaatgct 6600
agataccctt aataaacaca tcgaactcac taggaaaaga agtcgacggc ttcgctggga 6660
gtgcccaaga aagctaccct gccctcggcc atcagaagga tcttgtcaaa gagctcaaac 6720
agctcggaag acggctgatg aatggtcagg atgacggtct tgcccttctg cgacagcttc 6780
ttcagcacct ggacgacgct gtgggcggta aatgagtcca gtccggaggt gggctcatcg 6840
cagatcagaa gcggcggatc ggttagtgcc tcggaggcga atgccagacg cttcctttct 6900
ccgccggaca gacctttcac cctgccgggc acaccgatga tcgtgtgctg acatttgctg 6960
agcgaaagct cctggatcac ctgatccacg cgggccactc gctgccgata ggtcagatgt 7020

cgtggcatcc gcaccatggc ctggaaaatc aggtgttccc tggccgttag ggagccgata 7080
aagaggtcat cctgctggac ataggcgcac ctggcctgca tctccttggc gtccacaggt 7140
tggccattga gcagtcgcat cccggatggc gatacttgga tgccctgcgg cgatcgaaag 7200
gcaagggcat tcagcagggt cgtctttccg gcaccggaac tgcccatcac ggccaaaagt 7260
tcgcccggat aggccacgcc gcaaactgag tttcaaattg gtaattggac cctttattaa 7320
gatttcacac agatcagccg actgcgaata gaaactcacc gttcttgagc aaatgtttcc 7380
tgggcgccgg tatgtgtcgc tcgttgcaga atagtccgcg tgtccggttg accagctgcc 7440
gccatccgga gcccggctga ttgaccgccc caaagatgtc catattgtgc caggcatagg 7500
tgaggttctc ggctagttgg ccgctccctg aaccggagtc ctccggcgga ctgggtggca 7560
ggagcgtgcc gtagtttttg gcctgcccga agccctggtt aatgcagctc tgcgaagcgt 7620
ccgctgtcac cctgcaatga taggggatct caaatatcaa ctacaagcgt tatgctcatc 7680
taaccccgaa caaaacgaag tatcctacga agtaggttta tacttttatt tattttttgt 7740
gcatctagga tcagcttaaa atatctggtt gttatatttt ttgtaaaaaa gaatgtagtc 7800
gaaaatgaat gcctttagat gtcttgatca tgatatgatc ttaaaaattg tcttatatag 7860
cgagcacagc taccagaata atctgtttcg tgtcactatt tgtttgtgcg attgcggttt 7920
gggatttttg tgggtcgcag ttctcacgcc gcagacaatt tgatgttgca atcgcagttc 7980
ctatagatca agtgaactta agatgtatgc acatgtacta ctcacattgt tcagatgctc 8040
ggcagatggg tgtttgctgc ctccgcgaat taatagctcc tgatcctctt ggcccattgc 8100
cgggattttt cacactttcc cctgcttacc cacccaaaac caatcaccac cccaatcact 8160
caaaaaacaa acaaaaataa gaagcgagag gagttttggc acagcacttt gtgtttaatt 8220
gatggcgtaa accgcttgga gcttcgtcac gaaaccgctg acaaagtgca actgaaggcg 8280
gacattgacg ctaggtaacg ctacaaacgg tggcgaaaga gatagcggac gcagcggcga 8340
aagagacggc gatatttctg tggacagaga aggaggcaaa cagcgctgac tttgagtgga 8400
atgtcatttt gagtgagagg taatcgaaag aacctggtac ttcaaatacc cttggatcga 8460
agtaaattta aaactgatca gataag 8486

<210> 7
<211> 9100
<212> DNA
<213> Artificial Sequence

<220>
<223> Description of Artificial Sequence:-

<400> 7
ctagcttcga gcagctgaag cttgcatgcc tgcaggtcga ctctagagga tccactagta 60
acggccgcca gtgtgctgga attcggcttt gcttcgattc aatttaatcg tggcagtttg 120
cgagaatttc ggaatcggca tcagaggcga tctaccatgg cgcattaagt aagttggtaa 180
caaggattga accgcagcta aagcttttgt accatcagat ctgagctgaa gtacttcagc 240
cgcaccacca agcgaacaag tgatcccacc aagcaaaatg ccgtggtaat gggcaggaaa 300
acctacttcg gagtgccgga aagcaagagg cctcttcccg atcggctgaa catagtgctg 360
agcaccacac ttcaggaaag tgatctgccc aagggagtac tattgtgccc caatctcgag 420

acggccatga agatccttga ggagcagaac gaagtggaga acatttggat tgtgggcggt 480
agtggagtct acgaggaggc catggcctcg ccaaggtgtc accggctgta cattacccaa 540
ataatgcaga agttcgattg cgacaccttt ttccccgcga tccctgacag ctttcgagag 600
gtcgcgcccg attccgacat gccactgggt gtgcaggagg agaatggcat taaattcgag 660
tacaagattt tggagaagcc gaattctgca gatatccatc acactggcgg ccgcaataaa 720
gtagagggaa accgttgtgg tctccctata gtgagtcgta ttaaaggcct aattccagct 780
gagcgccggt cgctaccatt accagttggt ctggtgtcgg ggatccgtcg actaaggcca 840
aagagtctaa tttttgttca tcaatgggtt ataacatatg ggttatatta taagtttgtt 900
ttaagttttt gagactgata agaatgtttc gatcgaatat tccatagaac aacaatagta 960
ttacctaatt accaagtctt aatttagcaa aaatgttatt gcttatagaa aaaataaatt 1020
atttatttga aatttaaagt caacttgtca tttaatgtct tgtagacttt tgaaagtctt 1080
acgatacatt agtatctata tacatggttc attctacatt ctatattagt gatgatttct 1140
ttagctagta atacatttta attatattcg gctttgatga ttttctgatt ttttccgaac 1200
ggattttcgt agaccctttc gatctcataa tggctcattt tattgcgatg gacggtcagg 1260
agagctcgaa ttaacgggga tccgtcgacc tgcagcccaa gctttgcgta ctcgcaaatt 1320
attaaaaata aaactttaaa aataatttcg tctaattaat attatgagtt aattcaaacc 1380
ccacggacat gctaagggtt aatcaacaat catatcgctg tctcactcag actcaatacg 1440
acactcagaa tactattcct ttcactcgca cttattgcaa gcatacgtta agtggatgtc 1500
tcttgccgac gggaccacct tatgttattt catcatggtc tggccattct catcgtgagc 1560
ttccgggtgc tcgcatatct ggctctaaga cttcgggccc gacgcaagga gtagccgaca 1620
tatatccgaa ataactgctt gttttttttt ttaccattat taccatcgtg tttactgttt 1680
attgccccct caaaaagcta atgtaattat atttgtgcca ataaaaacaa gatatgacct 1740
atagaataca agtatttccc cttcgaacat ccccacaagt agactttgga tttgtcttct 1800
aaccaaaaga cttacacacc tgcatacctt acatcaaaaa ctcgtttatc gctacataaa 1860
acaccgggat atatttttta tatacatact tttcaaatcg cgcgccctct tcataattca 1920
cctccaccac accacgtttc gtagttgctc tttcgctgtc tcccacccgc tctccgcaac 1980
acattcacct tttgttcgac gaccttggag cgactgtcgt tagttccgcg cgattcggtg 2040
cggtatttca caccgcatat ggtgcactct cagtacaatc tgctctgatg ccgcatagtt 2100
aagccagccc cgacacccgc caacacccgc tgacgcgccc tgacgggctt gtctgctccc 2160
ggcatccgct tacagacaag ctgtgaccgt ctccgggagc tgcatgtgtc agaggttttc 2220
accgtcatca ccgaaacgcg cgagacgaaa gggcctcgtg atacgcctat ttttataggt 2280
taatgtcatg ataataatgg tttcttagac gtcaggtggc acttttcggg gaaatgtgcg 2340
cggaacccct atttgtttat ttttctaaat acattcaaat atgtatccgc tcatgagaca 2400
ataaccctga taaatgcttc aataatattg aaaaaggaag agtatgagta ttcaacattt 2460
ccgtgtcgcc cttattccct tttttgcggc attttgcctt cctgtttttg ctcacccaga 2520
aacgctggtg aaagtaaaag atgctgaaga tcagttgggt gcacgagtgg gttacatcga 2580
actggatctc aacagcggta agatccttga gagttttcgc cccgaagaac gttttccaat 2640
gatgagcact tttaaagttc tgctatgtgg cgcggtatta tcccgtattg acgccgggca 2700
agagcaactc ggtcgccgca tacactattc tcagaatgac ttggttgagt actcaccagt 2760
cacagaaaag catcttacgg atggcatgac agtaagagaa ttatgcagtg ctgccataac 2820
catgagtgat aacactgcgg ccaacttact tctgacaacg atcggaggac cgaaggagct 2880
aaccgctttt ttgcacaaca tgggggatca tgtaactcgc cttgatcgtt gggaaccgga 2940

gctgaatgaa gccataccaa acgacgagcg tgacaccacg atgcctgtag caatggcaac 3000
aacgttgcgc aaactattaa ctggcgaact acttactcta gcttcccggc aacaattaat 3060
agactggatg gaggcggata aagttgcagg accacttctg cgctcggccc ttccggctgg 3120
ctggtttatt gctgataaat ctggagccgg tgagcgtggg tctcgcggta tcattgcagc 3180
actggggcca gatggtaagc cctcccgtat cgtagttatc tacacgacgg ggagtcaggc 3240
aactatggat gaacgaaata gacagatcgc tgagataggt gcctcactga ttaagcattg 3300
gtaactgtca gaccaagttt actcatatat actttagatt gatttaaaac ttcattttta 3360
atttaaaagg atctaggtga agatcctttt tgataatctc atgaccaaaa tcccttaacg 3420
tgagttttcg ttccactgag cgtcagaccc cgtagaaaag atcaaaggat cttcttgaga 3480
tccttttttt ctgcgcgtaa tctgctgctt gcaaacaaaa aaaccaccgc taccagcggt 3540
ggtttgtttg ccggatcaag agctaccaac tctttttccg aaggtaactg gcttcagcag 3600
agcgcagata ccaaatactg tccttctagt gtagccgtag ttaggccacc acttcaagaa 3660
ctctgtagca ccgcctacat acctcgctct gctaatcctg ttaccagtgg ctgctgccag 3720
tggcgataag tcgtgtctta ccgggttgga ctcaagacga tagttaccgg ataaggcgca 3780
gcggtcgggc tgaacggggg gttcgtgcac acagcccagc ttggagcgaa cgacctacac 3840
cgaactgaga tacctacagc gtgagcattg agaaagcgcc acgcttcccg aagggagaaa 3900
ggcggacagg tatccggtaa gcggcagggt cggaacagga gagcgcacga gggagcttcc 3960
agggggaaac gcctggtatc tttatagtcc tgtcgggttt cgccacctct gacttgagcg 4020
tcgatttttg tgatgctcgt caggggggcg gagcctatgg aaaaacgcct tcttcttgaa 4080
ctcgggctcg gtgccagtat acctcaaatg gttgtcgtac ctctcatggt tccgttacgc 4140
caacgagggt ctgctgatta accaatgggc ggacgtggag ccgggcgaaa ttagctgcac 4200
atcgtcgaac accacgtgcc ccagttcggg caaggtcatc ctggagacgc ttaacttctc 4260
cgccgccgat ctgccgctgg actacgtggg tctggcccat gatgaaataa cataaggtgg 4320
tcccgtcgat agccgaagct taccgaagta tacacttaaa ttcagtgcac gtttgcttgt 4380
tgagaggaaa ggttgtgtgc ggacgaattt ttttttgaaa acattaaccc ttacgtggaa 4440
taaaaaaaaa tgaaatattg caaattttgc tgcaaagctg tgactggagt aaaattaatt 4500
cacgtgccga agtgtgctat taagagaaaa ttgtgggagc agagccttgg gtgcagcctt 4560
ggtgaaaact cccaaatttg tgatacccac tttaaagatt cgcagtggaa ggctgcacct 4620
gcaaaaggtc agacatttaa aaggaggcga ctcaacgcag atgccgtacc tagtaaagtg 4680
atagagcctg aaccagaaaa gataaaagaa ggctatacca gtgggagtac acaaacagag 4740
taagtttgaa tagtaaaaaa aatcatttat gtaaacaata acgtgactgt gcgttaggtc 4800
ctgttcattg tttaatgaaa ataagagctt gagggaaaaa attcgtactt tggagtacga 4860
aatgcgtcgt ttagagcagc agccgaatta attctagttc cagtgaaatc caagcatttt 4920
ctaaattaaa tgtattctta ttattatagt tgttattttt gatatatata aacaacacta 4980
ttatgcccac catttttttg agatgcatct acacaaggaa caaacactgg atgtcacttt 5040
cagttcaaat tgtaacgcta atcactccga acaggtcaca aaaaattacc ttaaaaagtc 5100
ataatattaa attagaataa atatagctgt gagggaaata tatacaaata tattggagca 5160
aataaattgt acatacaaat atttattact aatttctatt gagacgaaat gaaccactcg 5220
gaaccatttg agcgaaccga atcgcgcgga actaacgaca gtcgctccaa ggtcgtcgaa 5280
caaaaggtga atgtgttgcg gagagcgggt gggagacagc gaaagagcaa ctacgaaacg 5340
tggtgtggtg gaggtgaatt atgaagaggg cgcgcgattt gaaaagtatg tatataaaaa 5400
atatatcccg gtgttttatg tagcgataaa cgagtttttg atgtaaggta tgcaggtgtg 5460

taagtctttt ggttagaaga caaatccaaa gtctacttgt ggggatgttc gaaggggaaa 5520
tacttgtatt ctataggtca tatcttgttt ttattggcac aaatataatt acattagctt 5580
tttgaggggg caataaacag taaacacgat ggtaataatg gtaaaaaaaa aaacaagcag 5640
ttatttcgga tatatgtcgg ctactccttg cgtcgggccc gaagtcttag agccagatat 5700
gcgagcaccc ggaagctcac gatgagaatg gccagaccca cgtagtccag cggcagatcg 5760
gcggcggaga agttaagcgt ctccaggatg accttgcccg aactggggca cgtggtgttc 5820
gacgatgtgc agctaatttc gcccggctcc acgtccgccc attggttaat cagcagaccc 5880
tcgttggcgt aacggaacca tgagaggtac gacaaccatt tgaggtatac tggcaccgag 5940
cccgagttca agaagaagcc gccaaagagc aggaatggta tgataaccgg cggacccaca 6000
gacagcgcca tcgaggtcga ggagctggcg caggatatta gatatccgaa ggacgttgac 6060
acattggcca ccagagtgac cagcgccagg cagttgaaga agtgcagcac tccggcccgc 6120
agtccgatca tcggataggc aatcgccgtg aagaccagtg gcactgtgag aaaaagcggt 6180
aattcggcaa tcgttttgcc cagaaagtat gtgtcacagc gataaagtcg acttcgggcc 6240
tccctcataa aaactggcag ctctgaggtg aacacctaaa tcgaatcgat tcattagaaa 6300
gttagtaaat tattaatatg caaatgtatt ctaaacaaga cttacattta tcgtggcaaa 6360
gacgttttga aaggtcatgt tggtcaggaa gaggaagatg gctccgttga tattcatcac 6420
gcccacttgc gtgagttgtt ggcccaaaaa gatgaggcca atcaagatgg caaccatctg 6480
caaattaaaa tgttactcgc atctcattaa tattcatatc ttcaacatgt tcgcgagtta 6540
aatgaaattt atttattttc tgcaaaacta taaactatac atctcattga aaaaaactaa 6600
gaagggtgtg gaatcaggca attctaacta aaatctagcg aatttgtttc caagaattgt 6660
aagcgttata tcatttgttt ccactggaac cactcaccgt tgtctgaata agtcgcactt 6720
ttacgaggag tggttccttg agcaccgaca gccaggatcg ccacaggacc gcccggaact 6780
gcatgaacca ggtggccttg taggtgtacc cattctccgg ctgctccagt ggcttctcca 6840
aatttttggt ggccaacaac tgctccatat cccgggctac tttgctaata gcaaaattgt 6900
cgccatatct tggcgatccg atcacgggac tcgatctccc gtccgggcac aacggccaac 6960
acctgtacgt aaaagtccgc cggattgtag ttggtaggac actgggcacc cacgctggat 7020
aggagttgag atgtaatgta atgctagata cccttaataa acacatcgaa ctcactagga 7080
aaagaagtcg acggcttcgc tgggagtgcc caagaaagct accctgccct cggccatcag 7140
aaggatcttg tcaaagagct caaacagctc ggaagacggc tgatgaatgg tcaggatgac 7200
ggtcttgccc ttctgcgaca gcttcttcag cacctggacg acgctgtggg cggtaaatga 7260
gtccagtccg gaggtgggct catcgcagat cagaagcggc ggatcggtta gtgcctcgga 7320
ggcgaatgcc agacgcttcc tttctccgcc ggacagacct ttcaccctgc cgggcacacc 7380
gatgatcgtg tgctgacatt tgctgagcga aagctcctgg atcacctgat ccacgcgggc 7440
cactcgctgc cgataggtca gatgtcgtgg catccgcacc atggcctgga aaatcaggtg 7500
ttccctggcc gttagggagc cgataaagag gtcatcctgc tggacatagg cgcacctggc 7560
ctgcatctcc ttggcgtcca caggttggcc attgagcagt cgcatcccgg atggcgatac 7620
ttggatgccc tgcggcgatc gaaaggcaag ggcattcagc agggtcgtct ttccggcacc 7680
ggaactgccc atcacggcca aaagttcgcc cggataggcc acgccgcaaa ctgagtttca 7740
aattggtaat tggacccttt attaagattt cacacagatc agccgactgc gaatagaaac 7800
tcaccgttct tgagcaaatg tttcctgggc gccggtatgt gtcgctcgtt gcagaatagt 7860
ccgcgtgtcc ggttgaccag ctgccgccat ccggagcccg gctgattgac cgccccaaag 7920
atgtccatat tgtgccaggc ataggtgagg ttctcggcta gttggccgct ccctgaaccg 7980

gagtcctccg gcggactggg tggcaggagc gtgccgtagt ttttggcctg cccgaagccc 8040
tggttaatgc agctctgcga agcgtccgct gtcaccctgc aatgataggg gatctcaaat 8100
atcaactaca agcgttatgc tcatctaacc ccgaacaaaa cgaagtatcc tacgaagtag 8160
gtttatactt ttatttattt tttgtgcatc taggatcagc ttaaaatatc tggttgttat 8220
attttttgta aaaaagaatg tagtcgaaaa tgaatgcctt tagatgtctt gatcatgata 8280
tgatcttaaa aattgtctta tatagcgagc acagctacca gaataatctg tttcgtgtca 8340
ctatttgttt gtgcgattgc ggtttgggat ttttgtgggt cgcagttctc acgccgcaga 8400
caatttgatg ttgcaatcgc agttcctata gatcaagtga acttaagatg tatgcacatg 8460
tactactcac attgttcaga tgctcggcag atgggtgttt gctgcctccg cgaattaata 8520
gctcctgatc ctcttggccc attgccggga tttttcacac tttcccctgc ttacccaccc 8580
aaaaccaatc accaccccaa tcactcaaaa aacaaacaaa aataagaagc gagaggagtt 8640
ttggcacagc actttgtgtt taattgatgg cgtaaaccgc ttggagcttc gtcacgaaac 8700
cgctgacaaa gtgcaactga aggcggacat tgacgctagg taacgctaca aacggtggcg 8760
aaagagatag cggacgcagc ggcgaaagag acggcgatat ttctgtggac agagaaggag 8820
gcaaacagcg ctgactttga gtggaatgtc attttgagtg agaggtaatc gaaagaacct 8880
ggtacttcaa atacccttgg atcgaagtaa atttaaaact gatcagataa gttcaatgat 8940
atccagtgca gtaaaaaata aaaaaaaaat atgttttttt aaatctacat tctccaaaaa 9000
agggttttat taacttacat acatactaga attaattcct cgagcgaaat taatacgact 9060
cactataggg agaccacaac ggtttccctc tagtttattg 9100

## Description

The invention relates to the identification of target molecules in arthropods for insecticides or acaricides. Target genes of interest encoding such target molecules are especially genes that are active/inactive or show at least different expression profiles especially according to specific developmental stages/life cycles.

Due to the enormous damage that is still caused by insects on crops, wood, textiles as well as due to the transmission of diseases on or across human beings, animals and crops by various insects, the application of insecticides is still unavoidable. Insecticides are still an important part of integrated pest control and are heavily involved in order to guarantee/build up a stable or increased yield on harvests all over the world.

Moreover, the ecological as well as the economical demands on modern insecticides are permanently increasing and play an imporant role in order to develop new insecticides that are advantageous concerning toxicity, selectivity, needed quantities, residue levels, resistance, and the like when compared to known and/or applied today's insecticides. An important aspect in developing new insecticides is the search for new molecular target sites which are also able to serve as an integrated part of high throughput screening assays for the design of new and/or superior insecticides.

Concerning modern farming, feeding of insects on crop plants or related seeds is the major reason of crop damages caused by insect pests. Apart from direct killing of insect pests by e.g. neuro-active insecticides, crop damage can be reduced or avoided by chemical agents that are directed against specific developmental stages in order to be most efficient as well as more selective against beneficial insects and, consequently, these chemical agents have a greater chance to be environmentally friendly.

It is thus desirable for the control of insects to identify genes expressing proteins or being involved in other regulatory processes that are involved/active for example in various stages of insect development so that insecticides can be developed accordingly that exhibit a highly preferred or sole activity against such identified genes/regulatory processes of certain developmental processes/stages. Such genes/regulatory processes are best identified by determining their function through altering their expression levels.
Numerous techniques/examples are known from the literature all dealing with the down regulation or modulation of special genes, which can be either of endogenous or exogenous, i.e. viral source.

The most classical approach is the detection and selection of mutants which are generated spontaneously or which can be induced by manipulation for example by exposing individuals or cells to x-ray radiation or to chemicals. Especially in case of *Drosophila melanogaster,* a lot of mutants had been generated and established over decades by the application of these and related techniques. Nevertheless, these techniques can fail in cases where complex genetic pathways or regulation processes are involved in the metabolism of interest. In addition, for the target validation process it is important to monitor the effect of gradual decrease of gene expression in order to realisticly mimic the effect of a potential chemical inhibitor.

Beside these approaches, more and more new approaches are coming up - all of them based on genetic engineering techniques or the application of externally synthesized and applied naked nucleic acids, the latter must exhibit an improved resistance against enzymatic digestion.

It is obvious that the application of externally synthesized polynucleotides is strongly limited to such approaches where an uptake of these polynucleotides is possible which may be for example the case by using single cells, cell cultures or direct infusion into the bloodstream of a whole organism or the like.

The up to now by far most intensively described and applied technology in various organisms is the so called "antisense technology" (Akhtar and Agrawal (1997), Trends Pharmacol. Sci, 18, 12-18; Crooke et al. (1996), Ann. Rev. Pharmocol. Tocicol., 36, 107-129; Szymkowski (1996), Drug Discovery Today, 1, 415-428; Wagner and Flanagan (1997), Molecular Medicine Today, 3, 31-38. This technology allows - in theory - an interference of the delivered/present antisense RNA molecule with the naturally occuring sense RNA molecule - in most cases it is a messenger RNA (mRNA), but it may also be possible to design an antisense RNA that is directed, for example, against a viral RNA being present in a host cell. There are several aspects making such an antisense RNA working. Firstly, the sequence of the antisense RNA molecule must be at least over a certain number of bases complementary to the sense RNA towards it is directed. Secondly, the antisense RNA must be present inside the cell at the location where it should interact with the sense RNA to be targeted in at least stochiometric amounts, in most cases an excess up to a huge excess of such antisense RNA molecules may be necessary. There is always a problem to be kept in mind which can be explained by intramolecular secondary structure which can be formed by any RNA molecule and so it can be done by antisense RNA. Such intramolecular structures are strongly competing with intermolecular structures to be build up between the antisense and the sense RNA to be silenced down. Therefore, it is difficult to predict if a molecule that has been designed *in silico* will work inside a cell in a way as it should, based on its pure nuleotide composition.

In addition to this antisense technology as described above there are other approaches that are used in order to interfere in a highly specific manner with transcriptionally active genes, like it is the case with the so called "Triple Helix Approach" (Frank-Kamenetski and Mirkin (1995), Ann. Rev. Biochem., 64, 65-95; Gottesfeld et al. (1997), Nature, 202-205; Thuong and Hélène (1993), Angew. Chemie, 105, 697-723). This approach is based on an interaction at the DNA level. The application of this technology, which is the inner cell artificial formation of a triple helix on certain purin or pyrimidine nucleotiode stretches, depends on an efficient delivery of the triple helix forming polynucleotide to the target cell/organism. There are two major problems to be solved. Firstly, the polynucleotide must be protected against nuclease attacks, secondly, one must be able to direct the so protected polynucleotide to the target site, and thirdly, the interaction must be highly effective under physioligical conditions, like salt and pH conditions in the cellular environment.

Moreover, a technology has been described which is based on molecules that combine the binding of two short antisense regions and a catalytic activity. In case of such molecules - named as ribozymes in the literature (Cech (1986), Cell, 44, 207-210; Marr (1996), Drug Discovery Today, 1, 94-102; Scott and Klug (1996), Trends Biol. Sci., 21, 220-224) - it seems to be rather difficult to predict the level of activity of such molecules in the cellular environment. More specifically, problems of right sorting as well as the optimal intermolecular base pairing of such short antisense stretches which have to compete with the secondary and maybe tertiary structure of the ribozyme molecule as well as that of the target molecule arise and at the end a sufficient catalytic activity must be proven for each individual case separately.

Beside these approaches, there is another gene-specific approach known under the name "co-suppression", which means the ability of a sense transgene to interfere with the activity of an endogenous genetic locus. This phenomenon was described first in plants and it relates to the ability of transgenic plants to cause gene silencing of unlinked but homologous gene sequences. After that first detection in plants (Matzke and Matzke (1995), Plant Physiol. 107, 679-685; Angell and Baulcombe (1997), EMBO J. 16, 3675-3684; Kumagai et al. (1995), Proc. Natl. Acad. Sci. USA, 92, 1679-1683; Wassenegger et al. (1994), Cell, 76, 567-576), similar phenomena had been reported at least in two other organims: (1) in *Caenorhabditis elegans* (Fire et al. (1991), Development, 113, 503-514; Guo and Kemphus (1995), Cell, 81, 611-620; Fire et al. (1998), Nature, 391, 806-811), and (2) in *Drosophila melanogaster* (Kennerdell and Carthew (1998), Cell, 95, 1017-1026). The real scientific explanation of co-suppression is still missing but it may be possible that the interaction of the overexpressed transgene causing the reduction of the expression level of the endogenous gene is caused on the DNA level by , for example, hypermethylation of the endogenous gene leading to a suppression of the gene expression level of the endogenous gene, or the inference takes place on the RNA level via an RNA intermediate that had been synthesized at the transgene locus. Meanwhile, the latter theory is more supported by experiments with plant viruses.

In a number of publications, another technique has been described which is most often called as RNAi-technology (RNAinterference-technology). This technology is based on the generation of double-stranded RNA (dsRNA) molecules being identical to at least a part of the double-stranded DNA sequence of a gene which should be switched off or modulated (Bosher and Labouesse (2000), Nat. Cell. Biol., 2 E31-6). In case of using this technology in *Drosophila,* many applications of gene silencing based on dsRNA have been reported on transfection/injection assays (Hammond et al. (2000), Nature, 404, 293-296; Misquetta and Paterson (1999), Proc. Natl. Acad. Sci, USA, 96, 1451-1456; Kennerdal and Carthew (1998), Cell, 95, 1017-1026; Zamore et al. (2000), Cell, 101, 25-33).

A "double-stranded RNA (dsRNA)" molecule comprises a sense RNA fragment of a target gene and an antisense RNA fragment of the same target gene, which both comprise nucleotide sequences complementary to one another, thereby allowing the sense and antisense RNA fragments to pair and form a double-stranded molecule. "Complementary" refers to two nucleotide sequences which comprise antiparallel nucleotide sequences capable of pairing with one another upon formation of hydrogen bonds between the complementary base residues in the antiparallel nucleotide sequence.

"Antiparallel" refers herein to two nucleotide sequences paired through hydrogen bonds between complementary base residues with phosphodiester bonds running in the 5'->3' direction of one nucleotide sequence and in the 3'->5' direction of the other nucleotide sequence.

In addition to that, Kennerdell and Carthew reported (Kennerdell and Carthew (2000), Nature Biotechnology, 18, 896-898) that they were able to switch off certain reporter genes (i.g. IacZ) in transgenic flies by generating double-stranded RNA (dsRNA) via the transcription of an RNA molecule which generates its double-stranded RNA structure by folding back over a hairpin structure so to generate an extended hairpin-loop RNA.

As this way of generating dsRNA does not always result into a dsRNA of the structure as predicted, it was the problem to be solved to have an RNAi system working in a more predictable and reliable way in transgenic *Drosophila* flies than the system as described by Kennerdell and Carthew (Kennerdell and Carthew (2000), Nature Biotechnology, 18, 896-898) does. The main problem in using this hairpin loop approach is to overcome thermodynamic problems so that the majority of the built dsRNA really exists in the structure needed so to be active as a dsRNA molecule in the sense of silencing the gene of interest or modulating the gene expression of the gene of interest in an efficient an reliable way. These thermodynamic problems can only be overcome by always adapting a new hairpin building sequence in order to avoid any competing 1^{st} order reaction of dsRNA forming sequences. Even in case of *in silico* modelling of an optimised structure it is difficult to predict if this will be the structure existing in nature as this 1^{st} order reaction can result in one or more absolutely different dsRNA compared to the calculated one. Furthermore, when working in a transgenic organism any unintended activity or a certain degree of leakiness concerning the presence of the dsRNA must be avoided. Caused by this aspect/prerequisite, there is a strong requirement to develop a reliable and "safe" system, at least concerning dsRNA molecules which could modulate metabolic processes, steering elements or the like in a highly reliable way.

Beside this risk of imponderabilities in generating at least in a certain number of molecules the "wrong" which means a non-active dsRNA structure there is also a practical aspect why this approach of generating dsRNA molecules for screening several genes may not be the optimal one. This fact is caused by somewhat lengthy cloning procedures to be undertaken in case that several screening assays should be run or the optimal length or composition of the dsRNA should be determined. In addition to these arguments as listed above, it would be extremely powerful to establish a system wherein the switch on/switch off procedure steering the transcription of the selective dsRNA molecules is controlled in an easy and reliable way.

It has now been found that the selective transcription of dsRNA molecules comprising the nucleic acid information of a known endogenous gene - the target gene - or parts thereof inside a target organism in a way that dsRNA molecules are generated by transcribing RNA molecules and their respective complements in the same compartment from two promoters being located at the opposite sites of one dsDNA molecule can be used in a highly reproducible way in transgenic flies for the modulation of the transcription and/or expression of such endogenous genes. This can be done at least in exercising two various approaches.
Firstly, this can be done by using control elements that are located upstream or downstream at each of the two promoter elements and which can be triggered by certain molecules in a highly selective way so that the respective dsRNA is only present in cells in presence/absence of the triggering molecules. Such triggering molecules can either be delivered from outside like chemical compounds or can be encoded by endogenous genes which are activated by shifting environmental conditions like, for example, temperature.
Secondly, the presence of polymerase(s) (like for example but not limited to phage polymerases T7, T3, SP6 or the like) which are encoded in a transgenic organism and which expression is controlled by external parameters (like temperature, stress, or factors that can be influenced from outside) so that the generation of the dsRNA molecules correlates directly with the presence of the respective polymerases.
In order to have a better control concerning any leakage in case of the second approach, it may be necessary in certain cases to use two different promoters under the control of two different polymerases resulting in an absolutely safe system for the generation of dsRNA influenced transgenic organisms. The organisms to be tested must contain appropriate genes encoding the respective polymerase(s) in order to transcribe the dsRNA in a proper way from the delivered DNA sequence. Consequently, transgenic organisms must be created first containing the genes encoding the selected polymerase that are needed for running a proper transcription of the RNA molecules to be combined and so to result in a dsRNA molecule.
Such a safe system may be the system of choice in all cases where even a very little leakage may cause severe problems for the cell/cell culture/organism to be analysed.

This invention provides an insect organism, preferably *Drosophila,* such insect organism being genetically modified by the introduction of a DNA molecule comprising:
A.
   (a) DNA sequences of two identical or non-identical promoters in an opposite orientation with a multipurpose cloning site between these two,
   (b) at least one additional DNA sequence located between the promoter sequences of (a) and cloned into the multipurpose cloning site,
   (c) wherein such additional DNA sequences of (b) cloned into the multipurpose cloning site represent at least a part of a target gene of the target organism,
   (d) one or more further DNA sequences to enable the DNA molecule a stable integration into the genome of the host, and
B. further comprising at least one DNA sequence encoding a DNA depending RNA polymerase which specifically recognizes the promoters as given under (a).

Generally speaking, a "promoter" is any sequence region on a DNA molecule which is important for transcription of a downstream located DNA sequence. Promoters are recognised by specific DNA depending RNA polymerases and/or other DNA binding proteins.

"Transcription" is defined as any synthesis of an RNA molecule - the so called RNA transcript - facilitated by a DNA depending RNA polymerase and where the synthesised RNA is encoded by a DNA molecule that serves a template.

"Expression" is defined as the synthesis of any protein based on the translation of an RNA sequence of an RNA molecule into a polypeptide sequence.

A "multipurpose cloning site (mcs)" comprises a variable number of unique restriction sites for restriction enzymes that does not exist on the cloning vector so to enable the insertion of any DNA sequence of interest into the multipurpose cloning site. By choosing two separate restriction sites of such a multipurpose cloning site the skilled artisan is able to insert the DNA sequence of interest in a directed manner.

The term "target gene" comprises a gene of known function or is a gene whose function is unknown but whose total or patial nucleotide sequence is known.

Alternatively, function of a target gene and its nucleotide sequence are both unknown. The target gene is an integral part of the genome of the "target organism" which is the organism to be analysed. In case of gene families, one will be able by choosing the correct DNA sequence which is present in all such members of a gene family to switch off, reduce, or otherwise modulate the presence of all corresponding RNA transcripts, or by choosing specific DNA sequences being present at least on one of the DNA sequences belonging to such a gene family to switch off or reduce or otherwise modulate at least or especially only one of these corresponding transcripts.

Reference herein to a "target gene" is to be taken in its broadest context and includes:
(a) a classical genomic gene consisting of transcriptional and/or translational regulatory sequences and/or a coding region and/or non-translated sequences (i.e. introns, 5'- and 3'- untranslated sequences) and/or
(b) mRNA corresponding to the coding regions (i.e. exons) and 5'- and 3'-untranslated sequences of the gene; and/or
(c) a structural region corresponding to the coding region (i.e. exons) optionally further comprising untranslated sequences and/or a heterologous promoter sequence which constists of transcriptional and/or translational regulatory regions capable of conferring expression characteristics on said structural region.

DsRNA molecules containing a nucleotide sequence identical to a portion of the target gene are preferred for modulation but also dsRNA molecules with insertions, deletions, and single point mutations relative to the target sequence may also be used for modulation of such target genes. Sequence identity may be optimised by sequence comparison alignment algorithms known in the art (Gribskov and Devereux, Sequence Analysis Primer, Stockton Press, 1991, and references cited therein) and calculating the percent difference between the nucleotide sequences by, for example, the Smith-Waterman algorithm as implemented in BESTFIT software program using default parameters(e.g., University of Wisconsin Genetic Computer Group). Greater than 90% sequence identity, or even 100% sequence identity, between the moduationg dsRNA and the portion of the target gene is preferred, but also less than 90% sequence identity may be sufficient.

A "stable integration" means the integration of a foreign (this is a naturally non-occuring DNA-sequence) DNA sequence into the genome of the target organism.

Such DNA molecule additionally may comprise regulatory elements located upstream to the promoters which can be either influenced from outside by the application of chemical molecules outside the target organism or by changing environmental conditions, or which can be influenced by the presence or absence of endogenous factors. Alternatively, the presence or absence of the needed promoter specific polymerase(s) can be triggered directly by any changed condition, inernally or externally of the target organism.

Such DNA molecules may be transformed/transfected into a suitable host cell according to standard protocols of state of the art (Groebe, Chung, and Ho (1990), Nucleic Acids Res. 18, 4033; Voie and Cohen (1998), in: Cell Biology: A laboratory handbook, 2^{nd} Ed. Vol.3). Thus, in a further aspect, the invention provides a process for the preparation of any DNA molecule containing the various DNA sequences in order to establish a functional DNA molecule which is able to build up the intended dsRNA molecule in the right environment of a transgenic organism or single cells or cell cultures thereof.

The DNA molecule may be for example, a plasmid, provided with an origin of replication. The DNA molecule may contain one or more selectable marker genes, for example an ampicillin resistance gene in the case of a bacterial plasmid. DNA molecules may be used to transform/transfect a host cell, for example *E. coli, Spodoptera frugiperda* insect cells or *Drosphila* cells.

Any vector may be used which is suitable for transient expression in insect cells or insects or for transposition into the insect genome and for *in vivo* expression in transgenic insects and which contains two opposing promoters which are tolerant against promoter interference.

Furthermore, the invention provides host cells, comprising the DNA sequences and/or DNA molecules of present invention. The host cell can be a prokaryotic or eukaryotic cell transformed or transfected with one or more DNA molecules for the replication and/or expression of DNA sequences of the invention. The cells will be chosen to be compatible with said DNA molecule and may for example be bacterial, or insect cells. In case of insect cells that can be infected by a baculovirus are preferably employed.

Of course the described system of modulating the transcription/expression of endogeneous genes by using dsRNA generated in a way as described above is used primarily in insects, but it also can be adapted to any other organism in case that appropriate vectors are available so to generate the necessary transgenic organism carrying the respective polymerase(s) which are inducible by appropriate means. Such organisms may be but are not limited to fungus, yeast, plants, non-vertebrates as well as vertebrates. Consequently, the results obtained by using this technology may be applied in such organism for the detection of effects depending on the modulation of certain genes in the whole organism, which can be done in a highly selective way. This could lead to the identification of target molecules for chemical molecules to be used for plant control, animal control or for pharmaceutical drugs.

A highly preferred kind of promoters are prokaryotic promoters, like T7, T3, or SP6 promoters or the like (Dunn and Studier (1983), J. Mol. Biol., 166, 477-535 ; Chakraborty, Salvo, Majumder, Maitra, (1977), Biol. Chem., 252, 6485-6493; Krieg and Melton (1987) Methods Enzymol., 155 397-415). In case of using these kind of promoters the transgenic organisms to be analysed must contain at least one specific gene encoding at least one respective polymerase needed so to transcribe the DNA under the control of the respective promoter in a proper way. The presence of both, the gene(s) endoding the polymerase(s) of interest as well as the dsRNA encoding DNA sequence(s) in one organism can either be established by double-tranformation or - which is more preferred - by sexually crossing the organism containing the dsRNA encoding DNA with the polymerase(s) producing organism. Promoters and other expression signals may be selected to be compatible with the host cell for which the expression vector is designed. A suitable promoter for use is the inducible *lac*Z gene promoter, which is induced in the presence of Isopropylthiogalactosid (IPTG) (Hu and Davidson (1987), Cell , 48, 555-566). Other combinations are - for example but not limited to - any promoter which is controlled by the tetracycline repressor (Gossen and Bujard, (1992), Proc. Natl. Acad. Sci., USA, 89, 5547-5551), streptogramin-repressor (Fussenegger et al., (2000) Nature Biotechnol., 18, 1203-1208) or a inducible heat shock promoter.

The invention also provides a method of repressing, delaying or otherwise modulating the transcription and/or expression of at least one target gene in an insect cell or insect organism, comprising:
(a) the preparation of a DNA molecule characterized by the presence of DNA sequences of two identical or non-identical promoters in an opposite direction with a multipurpose cloning site between these two,
(b) wherein at least one additional DNA sequence is located between the promoter sequences and cloned into the multipurpose cloning site,
(c) wherein such additional DNA sequences of (b) represent at least a part of a target gene of the target organism,
(d) DNA sequences to enable the DNA molecule of a stable integration into the genome of the host,
(e) integration of the DNA molecule under (a) to (d) into the host genome,
(f) the transcription of the DNA sequence by the flanked promoters and wherein the transcribed DNA sequence is identical to an endogenous gene either as a whole or as a part of such an endogenous gene,
(g) wherein such individually transcribed RNA molecules are capable of forming non-covalently linked dsRNA molecules by any kind of intermolecular reactions,
(h) wherein such dsRNA molecules as formed according to (g) are able to modulate the presence of the protein encoded by the targeted gene, and
(i) wherein the modulation of protein activity is either visible at the phenotype of the organism or can be detected by any biochemical or enzymatic analysis.

The invention provides an assay for the detection of target genes and the corresponding target molecules which can be used for the identification of insecticidal targets. Such assays can be be done in appropriate single cells, cell cultures or whole transgenic organisms.

Such identified targets can be used, for example, for the identification of new chemical compounds that are interacting with at least one of these targets and hereby showing effects which are comparable to the effects that have been identified by modulating the gene expression of the gene encoding the protein molecule as being affected by such a chemical molecule.

The following examples are intended to illustrate the present invention without in any way restricting its scope.

The vectors having the SEQ ID NO: 5, SEQ ID NO: 6, and SEQ ID NO: 7 were deposited according to the Budapest Treaty at the DSMZ-Deutsche Sammlung von Mikroorganismen und Zellkulturen GmbH and obtained the catalogue number: DSM 13881 (SEQ ID NO: 5), DSM 13880 (SEQ ID NO: 6), and DSM 13882 (SEQ ID NO: 7).

### Example A: Generation of a vector for the expression of double-stranded RNA (dsRNA) in insects or insect cell culture

The minimum required sequence of the T7 promoter which makes it functional in any cellular environment is given by: TAATACGACTCACTATAGGGNGA (Imburgio, Rong, Ma, and McAllister, (2000), Biochemistry, 39, 10419-10430). RNA synthesised by the T7 promoter is, however, more stabile in *Drosophila* when it contains a stem-loop structure at its 5'-end (McCall and Bender, (1996), EMBO J., 15, 569-580). In order to provide this preferred secondary structure at the 5'-end, the T7 promoter transcribing naturally the so called mRNA 10 (messengerRNA 10) of the T7 phage and its correponding downstream sequences were used (nucleotide position 22881-position 22928; Acc# J02518). Although any vector can be used for the stepwise construction of a double T7 promoter plasmid in case of this example the vectors pSP72 (obtained by Promega) and pCaSpR-hs (Acc# U59056, C.S. Thummel, and V. Pirrotta, (1992): Dros. Info. Service 71, 150-150) have been selected.

The following steps were run for the preparation of a basic vector which can be used for every further cloning step so to integrate any DNA sequence of interest subsequently. Such cloned DNA sequence of interest can be transcribed in both directions so to result into the appropriate and predicted dsRNA:
- Vector pSP72 was digested with *Nhe*l and *Xho*l.
- Primers T7-1 up and T7-1 dw were mixed together at a final concentration of 1ug/30ul, boiled and cooled slowly to room temperature.
- The annealed primers were ligated into the vector using standard ligation procedure (Sambrook J et al, 1989 Molecular Cloning: A laboratory Manual 2nd Edition, Cold Spring Harbour Laboratory). The resulting vector is pSP72 T7-1 and it contains the newly inserted T7 promoter oriented towards the original T7 promoter.
- The originally contained T7 promoter of pSP72 T7-1 was replaced by the second mRNA10 T7 promoter by digesting the pSP72 T7-1 with *Bgl* II and *Hpa* I and ligating the annealed oligonucleotides T7-2 up and T7-2 dw using same standard ligation procedures as used above. The resulting plasmid is called "pSP72 T7-1+2" (SEQ ID NO: 5;) and it contains the two extended T7 promoters oriented towards each other, as well harbouring a multiple cloning site between.
- The double T7 cassette as generated in applying the steps as listed above was transferred as a *Xho* I/*Stu* I fragment to the P-element transposition vector pCaSpeR-hs which was digested with *Xho* I/*Stu* I as well. The resulting vector is called: pCaSpeR -T7>mcs<T7.

Applying these various cloning and sub-cloning steps lead to two different vectors which are identical in the promoter regions as well as the multi cloning site in between. As these vectors enable the activity in different cellular environment (pSP72 T7-1+2 to be used for testing the activity in single cells or tissue cultures, pCaSpeR -T7>mcs<T7 to be used in tissue culture and for Drosophila transformation) one will be able in using this vector combination so to check for identities/differences of effects that will be observed in single cells or tissue cultures when compared with a whole organism.

Sequences of T7-1 up, T7-1 dw, T7-2 up and T7-2 dw are listed below:
- T7-1 up (SEQ ID NO: 1; 5'-> 3'): 
TATCTCGAGCGAAATTAATACGACTCACTATAGGGAGACCACAACGGTTTCC
CTCTAGTTTATTGCTAGCT
- T7-1 dw (SEQ ID NO: 2; 5'-> 3'): 
TCGAAGCTAGCAATAAACTAGAGGGAAACCGTTGTGGTCTCCCTATAGTGA
GTCGTATTAATTTCGCTCGAGATATG
- T7-2 up (SEQ ID NO: 3; 5'-> 3'): 
AACAGGCCTTTAATACGACTCACTATAGGGAGACCACAACGGTTTCCCTCTA
CTTTATTGCGGCCGCA
- T7-2 dw (SEQ ID NO: 4; 5'-> 3'):

GATCTGCGGCCGCAATAAAGTAGAGGGAAACCGTTGTGGTCTCCCTATAGT
GAGTCGTATTAAAGGCC TGTT

The concept of producing dsRNA *in vivo* from a double promoter construct with the aim to interfere with the expression of genes expressed in that same cell can be proven in assays as given in the following examples (Examples B to C)

### Example B: Comparison of dsRNA activity directed to luciferase activity in tissue culture

Synthesis of dsRNA was done in order to influence the activity of the Firefly luciferase (Gelmini, Pinzani, and Pazzagli (2000), Methods Enzymol., 305, 557-576) that is expressed in tissue culture cells as well in whole transgenic *Drosophila* individuals. Although any other strategy for the insertion of target gene fragments between the two opposing promoters can be used, the pCaSpeR-T7>luc<T7 (SEQ ID NO: 6;) herein was constructed by inserting a *Xba* I/*Sph* I fragment from pGL3 promoter (obtainable from Promega) into a *Xba* I/*Sph* I linearised pCaSpeR-T7>mcs<T7.

In addition to the individual vector containing the two opposing promoters as well as the DNA sequence encoding for the dsRNA, a suitable RNA polymerase is required to transcribe the sense and antisense RNA which forms dsRNA *in vivo.* To produce dsRNA from a double T7 promoter construct, T7 RNA polymerase needs to be expressed in insect cells or insects. A suitable promoter is tissue or time specific if the expression of the endogenous gene is to be interfered only in certain organs or tissues or at defined developmental stages. If the candidate insecticide target is to be interfered in all expressing tissues in a inducible fashion a suitable promoter is the hSP70 promoter. Although any vector can be used for the stepwise construction of a T7 polymerase expressing plasmid there follows an example of how to achieve it in a proper way.
- A T7 RNA polymerase containing fragment was cut out of pLEW13 (Wirtz, Leal, Ochatt, and Cross, (1999), Mol. Biochem. Parasitol., 99,89-101) as a *Ssp* I/*Pst* I fragment and cloned into pHSP70 (Huynh and Zieler, (1999), J. Mol. Biol., 288, 13-20), linearized with *Pst* I and *Pvu* II. The resulting vector is called pHSP T7pol. This construct is suitable for a RNA interference assay in insect cell lines.
- The T7 RNA polymerase was cut from pHSP T7pol as a *Eco* RV/*Nhe* I fragment and ligated into a *Xba* I/*Eco* RI (blunt) linearized pCaSpR-hs vector. The resulting vector is called pCaSpeR-hs T7pol. This construct is suitable for a RNA interference assay in insect cell lines and as a transposition vector in Drosophila flies.

Down regulation of luciferase expression can be demonstrated in insect cells by cotransfection of a suitable luciferase expression vector (luciferase cloned into plZ (InVitrogen)), a double T7 promoter construct expressing ds luciferase RNA and a plasmid expressing T7 RNA polymerase. Transfection of insect cells is preferentially performed with Lipofectin (Gibco) or Fugen (Roche), according to manufacturers instructions. The equimolar combination of those three plasmids leads to more than 90% reduction of Firefly luciferase activity as compared to a combination of constructs where only single-stranded RNA (ssRNA), corresponding to Firefly luciferase, in sense or antisense orientation is produced or as compared to co-expressed *Renilla* luciferase (Grentzmann et al., (1998), RNA, 4, 479-86) or as compared to a combination of constructs where dsRNA corresponding to beta-Galactosidase or any other non specific sequence is produced. Increasing the amount of pCaSpeR-hs-T7>luc<T7 ( 0ug,0,01ug, 0,02ug, 0,05ug, 0,1ug, 0,2ug, 0,3ug, 0,4ug, 0,5ug, 0,6ug, 0,8ug, 1,0 ug to 1,5ug leads to a concentration dependent decrease of luciferase activity with a maximal reduction of 95% at 1,5 ug.

### Example C:

Beside the model system based on the Luciferase activity (see: Example B) a naturally occurring target molecule the to dihydrofolate reductase (DHFR, Hao et al. (1994). J. Biol. Chem., 269, 15179-15185) has been choosen as an interesting target side.
Therefore, the plasmid pCaSpeR-T7>dhfr<T7 (SEQ ID NO: 7;) was constructed by inserting a PCR fragment covering the entire Drosophila DHFR coding region (position 935 to position 1520 of DHFR gene (Acc# U06861)) into the vector pGemT (Promega). From there the DHFR fragment was transferred as a *Sph*I/*Not* I fragment into pSP72 T7-1+2. From there the DHFR fragment was transferred as a *Nhe* I/*Not* I fragment into pCaSpeR-T7>mcs<T7. For that method standard protocols (Sambrook et al., (1989), Molecular Cloning: A laboratory Manual 2nd Edition, Cold Spring Harbour Laboratory) were used. The efficiency of dsRNA interference against endogenous genes like DHFR is a highly powerful tool so to investigate the importance of that gene for insect survival or normal behaviour. Downregulation of DHFR is achieved by geneticly combining pCaSpeR- T7pol and pCaSpeR-T7>dhfr<T7. A by product of this cross are offspring containing only one of the two transgenes. Eggs or larvi that have been heat shocked for 1 hour at 37°C do not survive when they harbour both transgenes. Pupation or hatching is only observed in animals which contain only one transgene. This observation is recapitulated with independent transgenic lines and it clearly demonstrates that the system is powerful in order to demonstrate the effective reduction of an essential gene and its effects on the phenotypical behaviour when the system is activated just by shifting the temperature to a level where the T7 polymerase is activated and, subsequently, the dsRNA is transcribed so to reduce the presence of the endogenous DHFR in the flies.
pCaSpeR-hs T7pol is suitable to induce production of dsRNA via a temperature shift from ambient 25°C to 37°C in that T7 RNA polymerase is not expressed at lower temperatures but strongly induced after heat shock. Induction of T7 RNA polymerase expression can however be achieved by any other suitable induction system like the tetracyclin induction system.

## Claims

1. An insect organism being genetically modified by the introduction of an isolated DNA molecule comprising:
A.
(a) DNA sequences of two identical or non-identical promoters in an opposite orientation with a multipurpose cloning site between these two,
(b) at least one additional DNA sequence located between the promoter sequences of (a) and cloned into the multipurpose cloning site,
(c) wherein such additional DNA sequences of (b) cloned into the multipurpose cloning site represent at least a part of a target gene of the target organism, and
(d) one or more further sequences to enable the DNA molecule a stable integration into the genome of the host, and
B. further comprising at least one DNA sequence encoding a DNA depending RNA polymerase which specifically recognizes the promoters as given under (a).

2. A DNA molecule according to claim 1A, wherein the promoter sequences are selected from the bacterial promoters SP6, T7, T3, and derivatives thereof still exhibiting the transcriptional activity of a promoter that is recognized by its natural SP6, T3 or T7 polymerase.

3. A DNA molecule as claimed in claim 2, wherein the promoter sequences are identical concerning the recognition by one specific polymerase.

4. A DNA molecule as claimed in claim 2, wherein the promoter sequences are non-identical concerning the recognition by a specific polymerase.

5. A transgenic insect organism as claimed in claim 1, which is *Drosophila.*

6. A method for repressing, delaying or otherwise modulating the transcription and/or the expression of at least one target gene in an insect cell or an insect organism, comprising:
(a) the preparation of a DNA molecule **characterized by** the presence of DNA sequences of two identical or non-identical promoters in an opposite direction with a multipurpose cloning site between these two,
(b) wherein at least one additional DNA sequence is located between the promoter sequences and cloned into the multipurpose cloning site,
(c) wherein such additional DNA sequences of (b) represent at least a part of a target gene of the target organism,
(d) DNA sequences to enable the DNA molecule of a stable integration into the genome of the host,
(e) integration of the DNA molecule under (a) to (d) into the host genome,
(f) the transcription of the DNA sequence by the flanked promoters and wherein the transcribed DNA sequence is identical to an endogenous gene either as a whole or as a part of such an endogenous gene,
(g) wherein such individually transcribed RNA molecules are capable of forming non-covalently linked dsRNA molecules by any kind of intermolecular reactions,
(h) wherein such dsRNA molecules as formed according to (g) are able to modulate the presence of the protein encoded by the targeted gene, and
(i) wherein the modulation of protein activity is either visible at the phenotype of the organism or can be detected by any biochemical or enzymatic analysis.

7. Use of a protein that has been identified by the method according to claim 6 in sceening assays for insecticidal compounds.
